# FASCICULE DE BREVET EUROPEEN

(11) **EP 0 661 035 B1**
(45) Date de publication et mention de la délivrance du brevet: **07.08.1996**
(21) Numéro de dépôt: 94402885.1
(22) Date de dépôt: 14.12.1994
(51) Int. Cl.: A61K 7/00

(54) **Composition cosmétique pour le traitement simultané des couches superficielles et profondes de la peau**
Kosmetisches Mittel zur gleichzeitigen Behandlung von Hautoberschichten und von Hauttiefschichten
Cosmetic composition for the simultaneous treatment of the superficial skinlayers and the deep skinlayers

(30) Priorité: 30.12.1993 FR 9315863
(43) Date de publication de la demande: 05.07.1995
(73) Titulaire: L'OREAL, F-75008 Paris (FR)
(72) Inventeur: Ribier, Alain, F-75001 Paris (FR); Simonnet, Jean-Thierry, F-75011 Paris (FR)
(74) Mandataire: Lhoste, Catherine

(56) Documents cités:
- EP-A- 0 559 502
- WO-A-93/15708
- FR-A- 2 408 387
- SOAP, COSMETICS, CHEMICAL SPECIALTIES, vol.69, no.7, 793, US page 77 'formulation ideas'
- INTERNATIONAL JOURNAL OF PHARMACEUTICS, vol.62, no.1, 1990, NL pages 75 - 79 GABRIJELCIC ET AL. 'evaluation of liposomes as drug carriers into the skin by one-dimentional epr imaging'

## Description

La présente invention a pour objet une composition pour le traitement cosmétique ou dermatologique des imperfections ou affections de la peau, y compris le cuir chevelu. Elle se rapporte plus particulièrement à une composition de ce type comprenant au moins un actif véhiculé par au moins deux catégories distinctes de vésicules lipidiques.

L'invention se rapporte aussi à une utilisation de cette composition pour le traitement cosmétique de la peau, à l'utilisation de cette composition pour la préparation d'une pommade destinée au traitement dermatologique de la peau et à un procédé de traitement cosmétique de la peau.

On connait de nombreux exemples de compositions cosmétiques ou dermatologiques destinées au traitement de la peau présentant un ou des actifs adaptés au traitement de la peau, encapsulés dans des vésicules ou sphérules lipidiques (appelées aussi liposomes).

On entend par vésicules ou sphérules lipidiques des particules formées d'une membrane constituée par un ou plusieurs feuillets concentriques, ces feuillets comportant une ou plusieurs couches bimoléculaires de lipides amphiphiles encapsulant une phase aqueuse. La phase aqueuse peut contenir des substances actives hydrosolubles et les couches bimoléculaires de lipides amphiphiles peuvent contenir des substances actives lipophiles.

Ces sphérules ont généralement un diamètre moyen compris entre 10 et 5000 nanomètres.

Parmi les nombreux documents publiés concernant cette matière, on peut citer le certificat d'addition français 2408387 qui décrit une composition à base de dispersions aqueuses de sphérules lipidiques ioniques ou non ioniques encapsulant au moins une substance active. Plus précisément, ce document décrit des compositions contenant au moins deux dispersions de sphérules contenant des actifs différents dans le but d'obtenir un système mixte, c'est-à-dire un système où l'on associe une première dispersion de sphérules contenant une première catégorie de substance active à une seconde dispersion de sphérules contenant une autre catégorie de substance active, qui permet aux deux catégories de substances d'agir simultanément au moment du traitement et d'obtenir éventuellement un effet synergique qui ne se produirait pas si l'on faisait agir successivement et séparément ces deux catégories de substances.

La demanderesse a maintenant mis au point des compositions cosmétiques ou dermatologiques permettant l'action simultanée de deux actifs différents et permettant, de plus, à ces actifs d'agir dans des zones différentes de la peau, c'est-à-dire dans les couches superficielles et dans les couches profondes de la peau, augmentant de ce fait très nettement l'efficacité de ces compositions et l'effet complémentaire ou synergique des actifs mis en oeuvre.

La demanderesse a également mis au point des compositions cosmétiques ou dermatologiques permettant au même actif d'agir simultanément dans les couches superficielles et dans les couches profondes de la peau, assurant un traitement plus complet et donc plus efficace du désordre dont elle souffre.

Or, il se trouve de nombreuses situations dans lesquelles les désordres de la peau concernent à la fois une atteinte des couches superficielles de la peau et une altération conjointe des couches plus profondes de celle-ci.

Il est bien connu que la peau est constituée de couches superficielles, le *Stratum Corneum*, et de couches profondes, l'épiderme vivant et le derme. Or, on ne savait pas dans l'art antérieur délivrer spécifiquement tel actif dans les couches superficielles et, simultanément, le même ou tel autre actif dans les couches profondes.

La présente invention a pour objet une composition cosmétique ou dermatologique pour le traitement simultané des couches superficielles et profondes de la peau, telle que définie dans les revendications 1 et 2.

Selon un mode de réalisation particulier, les actifs contenus dans la première dispersion de vésicules et dans la deuxième sont les mêmes.

La demanderesse a utilisé un moyen de tri des vésicules permettant à l'homme de l'art de sélectionner aisément les vésicules lipidiques aptes à véhiculer l'actif dans les couches profondes de la peau, appelées vésicules de profondeur, et celles aptes à véhiculer l'actif dans les couches superficielles de la peau, appelées vésicules de surface.

Ce tri s'effectue sur la base de la constante de diffusion D d'une sonde introduite dans les vésicules. Cette sonde est l'ASL [l'iodure de N-(1-oxyl-2,2,6,6-tétraméthyl-4-pipéridinyl)-N-diméthyl-N-hydroxyéthylammonium] de formule : 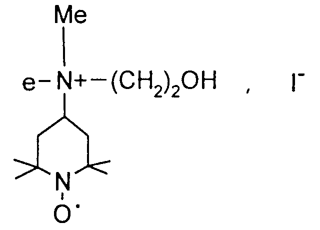 Les vésicules pour lesquelles la constante de diffusion D de la sonde dans le *Stratum Corneum* est > 1.10⁻⁷ cm² s⁻¹ sont les vésicules aptes à pénétrer dans les couches profondes de la peau.

Les vésicules pour lesquelles la constante de diffusion D de la sonde dans le *Stratum Corneum* est < 1.10⁻⁷ cm² s⁻¹ sont les vésicules aptes à véhiculer l'actif dans les couches superficielles de la peau.

Les vésicules de la première catégorie, dites de profondeur, sont en général à l'état fluide à température ambiante (autour de 20 °C), et celles de la seconde catégorie, dites de surface, sont en général à l'état gélifié à température ambiante. Le moyen de reconnaître l'état des vésicules consiste à déterminer la température de transition de phase (lamellaire fluide-gel) du lipide principal constituant leur membrane, par analyse thermique différentielle (ATD).

D'autres caractéristiques de ces vésicules sont en relation avec leur aptitude à délivrer l'actif plus ou moins en profondeur dans la peau. C'est en particulier le cas du taux d'encapsulation.

Le glucose est un marqueur classiquement utilisé pour ce type de détermination (cf. notamment Liposomes a practical approach de R.R.C. New, IRL Press (1990), p. 125-136).

Le taux d'encapsulation est exprimé par le volume de solution de glucose, encapsulée dans les vésicules, mesuré en µl par rapport à l'unité de poids (mg) des lipides constituant la membrane. Ce taux d'encapsulation est déterminé immédiatement après l'étape de séparation du glucose libre et du glucose encapsulé (Tₒ) ainsi que vingt-quatre heures après cette séparation (T₂₄ₕₑᵤᵣₑₛ).

La différence entre ces 2 déterminations successives illustre la perméabilité des vésicules vis-à-vis du glucose encapsulé, ce qu'on peut encore appeler leur potentiel d'encapsulation.

La première catégorie de vésicules (délivrant l'actif dans les couches profondes de la peau) présente un fort potentiel d'encapsulation des petites molécules hydrosolubles classiquement modélisées par le glucose, ce potentiel d'encapsulation se maintenant au moins 24 heures. La seconde catégorie de vésicules (délivrant l'actif dans les couches superficielles de la peau) ne retient pas le glucose à l'état encapsulé pendant le même temps.

Les lipides principaux constituant les vésicules de la première catégorie (actif délivré en profondeur) comportent au moins une chaîne grasse linéaire et saturée de longueur allant de 16 à 30 atomes de carbone tels que les phospholipides hydrogénés (de plantes ou d'oeuf), les phospholipides synthétiques saturés tels que la dipalmitoylphosphatidylcholine, les alkyléthers ou alkylesters de polyols à une, deux ou trois chaînes grasses par molécule. Ces lipides sont utilisés seuls ou en mélange.

Les lipides principaux constituant les vésicules de la seconde catégorie (actif délivré en surface) sont choisis en particulier dans le groupe comprenant des lipides ioniques tels que notamment les phospholipides naturels à base de plantes ou d'oeuf, contenant des chaînes grasses insaturées ayant de 16 à 30 atomes de carbone ; des lipides non ioniques tels que les alkyléthers ou alkylesters de polyols comportant une ou plusieurs chaînes grasses par molécule, dont au moins une chaîne grasse de longueur inférieure à 16 atomes de carbone, tels que le lauryl polyglycéryl-6-cétéaryl glycol éther, décrit en détail dans la demande de brevet FR 92-09603 déposée par L'Oréal.

On peut de façon connue incorporer dans la phase lipidique constituant la membrane lipidique des vésicules, au moins un additif choisi dans le groupe formé des stérols (phytostérols, cholestérol, phytostérols polyoxyéthylénés) ; des alcools, diols et triols à longue chaîne (phytanetriol), des amines à longue chaîne et leurs dérivés ammonium quaternaire ; des esters phosphoriques d'alcools gras et leurs sels alcalins (Na, K) tels que le dicétylphosphate, le dicétylphosphate de sodium, les alkylsulfates (cétylsulfate de sodium), les sels alcalins du cholestérol sulfate ou du cholestérol phosphate, le sel de sodium de l'acide phosphatidique, les lipoaminoacides et leurs sels tels que les acylglutamates de sodium.

On peut citer comme exemple de vésicules de la première catégorie (délivrant l'actif dans les couches profondes de la peau) les vésicules obtenues à partir des lipides suivants (nom CTFA) :
- A/ cholestérol / lipoaminoacide caséique notamment dans un rapport pondéral 45/45/10 (où A est un cétyléther de triglycéryle commercialisé par la société Chimex sous le nom Chimexane NL) ;
- B/ cholestérol / dicétylphosphate notamment dans un rapport pondéral 60/35/5 (où B est un mélange de mono, di et tricétyléther de triglycéryle commercialisé par la société Chimex sous le nom Chimexane NT) ;
- Span 40 (de chez Ici ou Sorbitan palmitate)/ cholestérol / acylglutamate de sodium (vendu par la Société Ajinomoto sous le nom HS 11) notamment dans un rapport pondéral 47,5/47,5/5 ;
- Stéarate de PEG 8 / cholestérol / acylglutamate de sodium avec notamment un rapport pondéral 47,5/47,5/5 (où le stéarate de PEG 8 est le polyéthylène glycol à 8 motifs d'oxyde d'éthylène commercialisé par la société Unichema sous le nom Stéarate PEG 400) ;
- Stéarate de PEG 8 / cholestérol / phytanetriol / acylglutamate de sodium avec notamment un rapport pondéral 47,5/20/27,5/5 ;
- Lécithine hydrogénée / phytostérol polyoxyéthyléné à 5 motifs d'oxyde d'éthylène notamment dans un rapport pondéral 60/40 ;
- distéarate de méthylglucose polyoxyéthyléné à 20 motifs d'oxyde d'éthylène / cholestérol / acylglutamate de sodium notamment dans un rapport pondéral 45/45/10 (le distéarate étant par exemple vendu sous le nom Glucam E 20 distéarate par Amerchol) ;
- A / cholestérol / dicétylphosphate avec notamment un rapport pondéral 47,5/47,5/5 ;
- distéarate de diglycéryle (par exemple celui vendu par Nihon sous le nom Emalex DS G2) /cholestérol / acylglutamate de sodium dans un rapport pondéral 45/45/10 ;
- mono et distéarate de saccharose (par exemple celui vendu par Grillo sous le nom Grilloten PSE 141 G) / cholestérol / acylglutamate de sodium, notamment dans un rapport pondéral 45/45/10 ;
- tristéarate de tétraglycéryle (par exemple celui vendu par Nikkol sous le nom Tetraglyn 3S) / cholestérol / acylglutamate de sodium, notamment dans un rapport pondéral de 45/45/10.

On peut citer comme exemples de vésicules de la deuxième catégorie (délivrant l'actif dans les couches superficielles de la peau) les vésicules obtenues à partir des lipides suivants :
- Lécithine de tournesol ;
- Natipide II (lécithine de soja / éthanol / eau dans un rapport pondéral 60/20/20 commercialisé par Nattermann) ;
- C (lécithine de soja / cholestérol / propylène glycol dans un rapport pondéral 40/30/30 commercialisé par Nattermann sous le nom NAT 50 PG) ;
- D / dimyristylphosphate notamment dans un rapport pondéral 95/5 (où D est un éther de lauryl polyglycéryl-6-cétéarylglycol commercialisé par Chimex sous le nom Chimexane NS).

Le tableau I ci-après donne pour certaines vésicules obtenues à partir des lipides ci-dessus, la constante de diffusion D de l'ASL dans le *Stratum Corneum* et dans l'épiderme/derme ainsi que le taux d'encapsulation du glucose, et la température de transition de phase du lipide principal constituant la membrane. La constante de diffusion a été mesurée pour une concentration en ASL encapsulé de 0,35 % en poids par rapport au poids total de la composition.

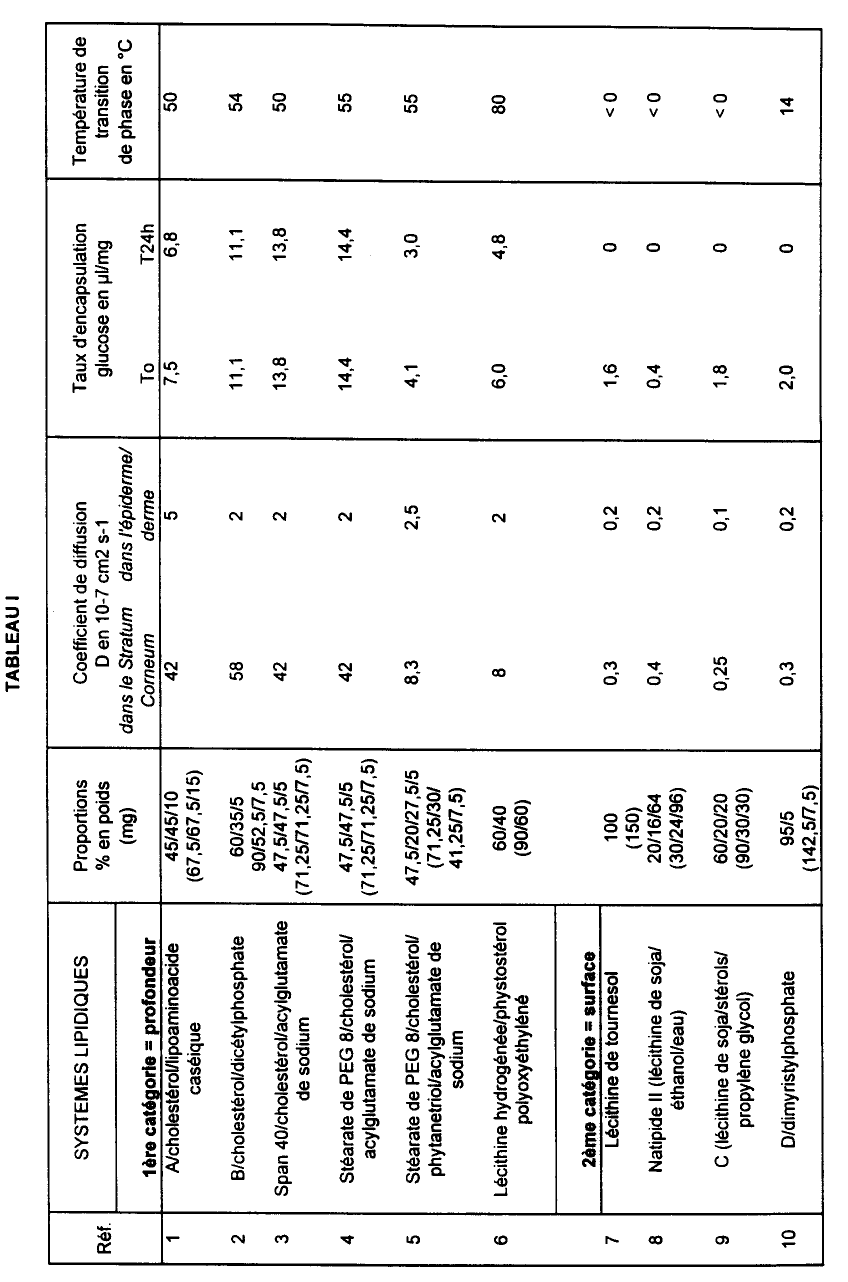

La mesure de la constante de diffusion D s'effectue par combinaison de deux méthodes utilisant une sonde paramagnétique, l'ASL : la résonance paramagnétique électronique (RPE) unidimensionnelle et périodique d'une part et l'imagerie cinétique RPE d'autre part. Ces deux méthodes sont décrites respectivement dans les articles International Journal of Pharmaceutics, 62 (1990) p. 75-79, Elsevier de V. Gabrijelcic et al. "Evaluation of liposomes as drug carriers into the skin by one-dimensional EPR imaging" et Periodicum Biologorum vol. 93, n° 2, p. 245-246, (1991) de V. Gabrijelcic et al. "Liposome entrapped molecules penetration into the skin measured by nitroxide reduction kinetic imaging".

La mesure du taux d'encapsulation s'effectue comme décrit dans le document RRC New cité précédemment et celle de la température de transition de phase comme décrit ci-dessus.

Les compositions objet de la présente invention trouvent leur application dans un grand nombre de traitements cosmétiques ou dermatologiques de la peau, y compris le cuir chevelu, comme par exemple : l'hydratation, la nutrition, la protection, le raffermissement, l'antiâge, l'antirides, l'amincissement, la dépigmentation, l'antiacné, et les traitements des mycoses, des dermites et du psoriasis. Aussi, l'invention a encore pour objet une utilisation de la composition définie ci-dessus pour le traitement cosmétique de la peau, du visage et/ou du corps ainsi que pour la préparation d'une pommade destinée au traitement des maladies de peau. L'invention a encore pour objet un procédé de traitement cosmétique de la peau, consistant à appliquer la composition ci-dessus sur la peau.

Les actifs encapsulés dans les vésicules de première et de deuxième catégories dépendent, bien entendu, de l'application visée.

Avantageusement, on utilise simultanément plusieurs actifs dans chaque catégorie de vésicules, présentant la même fonction et/ou conférant à la peau, en surface et en profondeur, le même type d'effet ; les actifs de surface et de profondeur sont donc complémentaires.

En particulier, les actifs des première et seconde catégories de vésicules sont, soit des actifs antiâge, soit des actifs antirides, soit des hydratants ou humectants, soit des actifs amincissants, soit des actifs dépigmentants, soit des actifs antiradicaux libres (espèces radicalaires de l'oxygène), soit des actifs anti-irritation, soit des actifs nutritifs, soit des actifs protecteurs, soit des actifs restructurants, soit des actifs raffermissants, soit des actifs antiacnéïques, soit des actifs exfoliants, soit des actifs émollients, soit encore des actifs traitants des maladies de peau comme les mycoses, les dermites, le psoriasis, etc...

A titre d'exemple pour l'hydratation, on associe aux vésicules de surface un ou plusieurs humectants (ou hydratants) tels que la glycérine, l'urée et aux vésicules de profondeur un ou plusieurs agents précurseurs de la biosynthèse des protéines structurales tels que l'hydroxyproline, les peptides de collagène, etc ...

Pour l'amincissement, on associe par exemple au moins un agent kératolytique ou un alpha hydroxy-acide tel que l'acide salicylique ou n-octanoyl - 5 salicylique encapsulé dans les vésicules de surface à au moins à un agent liporégulateur tel que la caféine, encapsulé dans les vésicules de profondeur.

Pour la dépigmentation, on combine par exemple au moins un agent kératolytique, encapsulé dans les vésicules de surface, à au moins un dépigmentant tel notamment qu'un inhibiteur de tyrosinase (acide kosique), encapsulé dans les vésicules de profondeur.

Dans le cas où l'on vise une protection vis-à-vis des méfaits des espèces radicalaires ou singulet de l'oxygène, on peut par exemple associer un agent antiradicaux ROO^{·} (vitamine E) ou antioxygène singulet pour le traitement en surface à un antiradicaux libres O2^{·} (superoxyde dismutase) et OH^{·} (sucre, caféine) pour le traitement en profondeur.

De même pour une action antiâge, on peut associer des humectants (lactate de sodium), des filtres antisolaires, des alpha hydroxy-acides (acides de fruit) ou des restructurants de surface, encapsulés dans des vésicules de surface, à des peptides (extraits peptidiques de soja notamment), des enzymzes de réparation de l'ADN, des agents protecteurs vasculaires, ou des phospholipides extraits du poulpe, riches en oligo-éléments et en acides gras polyinsaturés C20 -4, 20-5, 22-4, 22-5, 22-6, encapsulés dans des vésicules de profondeur.

Si l'on vise la nutrition de la peau, on peut par exemple associer au moins un actif tel des sucres, des acides gras pour une action en surface, à au moins un actif tel que notamment des vitamines et des acides aminés pour une action en profondeur.

Si l'on envisage le traitement des mycoses, il est possible d'associer des actifs pour agir en surface comme par exemple des exfoliants à des agents antimycosiques pour agir en profondeur. Les actifs peuvent représenter de 0,02% à 5% du poids total de la composition. Les compositions selon l'invention peuvent présenter toutes les formes galéniques normalement utilisées pour une application topique telles que les gels aqueux, les émulsions, les lotions, les pommades, les sérums et plus particulièrement les gouttelettes d'huile dispersées par les vésicules telles que décrites dans les brevets français FR-A-2485921 et FR-A-2490504.

De façon connue, dans les compositions de l'invention on peut trouver en plus des vésicules, une huile végétale, minérale, siliconée ou synthétique, dispersée dans une phase aqueuse et également des adjuvants hydrophiles comme les gélifiants, les conservateurs, les opacifiants, des adjuvants lipophiles comme des parfums, des pigments et des charges comme décrit dans les brevets français ci-dessus. L'huile dispersée peut représenter de 2 % à 40 % en poids par rapport au poids total de la composition et les adjuvants peuvent représenter, au total, de 0,1 % à 10 % en poids.

L'invention a aussi pour objet l'utilisation de la composition définie précédemment et un procédé pour le traitement antiâge, antirides, dépigmentant, nutritif, amincissant, hydratant et/ou antiacnéique de la peau ; ce procédé consiste à appliquer sur la peau, localement ou sur tout le visage et/ou le corps, une composition telle que définie ci-dessus.

L'invention se rapporte encore à l'utilisation de cette composition pour la préparation d'une pommade destinée aux traitements des maladies de peau tels que les traitements antimycosique, antidermite et antipsoriasis de la peau.

D'autres caractéristiques et avantages de l'invention ressortiront mieux de la description qui suit, donnée à titre illustratif et non limitatif.

### A) Obtention de vésicules lipidiques renfermant l'ASL

Les lipides constitutifs de la paroi des vésicules sont pesés et dissous dans 10 ml de méthanol. La solution alcoolique est alors transvasée dans un flacon rond de 50 ml à col rodé, que l'on place ensuite sur un évaporateur rotatif, de telle façon que le contenu soit thermostaté à la température de 30 °C. L'évaporation est poussée jusqu'au dépôt d'un film sec de lipides sur les parois du flacon.

3 ml d'une solution aqueuse 0,01 molaire d'ASL sont alors ajoutés dans le ballon qui est ensuite secoué à la main pendant environ 10 minutes, soit à la température ambiante (20 °C) pour les vésicules du tableau I référencées 7 à 10, soit à la température de 50 °C pour les vésicules référencées 1 à 6 du tableau I. On laisse alors le milieu s'équilibrer à température ambiante, pendant 2 heures, puis on place la dispersion dans un sac de dialyse et au contact de 500 ml d'eau distillée. La dialyse s'effectue pendant une nuit. Après une nuit, l'eau est changée et la dialyse est poursuivie pendant 4 heures supplémentaires.

Un fil de coton de 0,3 mm d'épaisseur est alors mis à tremper dans la dispersion de vésicules puis mis au contact d'une coupe de peau provenant d'une oreille de porc fraîchement récupérée dans un abattoir destiné à l'approvisionnement alimentaire.

L'échantillon d'oreille prélevé est rincé à l'eau et découpé en tranches de 1 mm d'épaisseur, 5 mm de large et 10 mm de long puis placé dans une cellule de maintien. Les mesures de diffusion de l'ASL dans la peau sont effectuées dans les 24 heures suivant le prélèvement de peau.

### B) Obtention de la composition cosmétique

### 1° Obtention des vésicules de première catégorie (diffusant en profondeur)

On prépare les vésicules (de profondeur) selon une méthode usuelle de co-fusion des constituants (voir tableau I) de la membrane choisis. Ainsi, on fond le constituant membranaire ayant le point de fusion T_{f} le moins élevé ; on ajoute les autres constituants membranaires puis on homogénéise sous agitation moyenne et enfin on hydrate partiellement en maintenant la température de fusion T_{f} définie ci-dessus.

A la pâte obtenue, on ajoute une solution aqueuse d'au moins un premier actif, pour le traitement en profondeur. Une turbine est actionnée pendant 1h 30 pour bien hydrater, en maintenant la température T_{f}. On rajoute au milieu réactionnel un ou plusieurs autres actifs pour le traitement en profondeur, on homogénéise et diminue la température du milieu jusqu'à la température ambiante (20 °C).

### 2° Obtention de vésicules de seconde catégorie (diffusant en surface)

On introduit à température ambiante (20 °C) et par simple agitation une solution aqueuse d'un (ou plusieurs) second actif pour le traitement en surface dans le mélange choisi de constituants devant former la membrane des vésicules de surface (voir tableau I). On obtient ainsi des vésicules de surface encapsulant le second actif de surface.

### 3° Obtention de la composition "double liposomes"

Au milieu contenant les vésicules de profondeur, on ajoute la phase grasse de la composition et on la disperse (à température ambiante) sous agitation. On mélange alors le milieu réactionnel obtenu à celui contenant les vésicules de surface. Eventuellement, on ajoute alors les adjuvants tels que des conservateurs, un gélifiant que l'on peut neutraliser si nécessaire par une base (triéthanolamine ou soude) et des parfums, etc...

Le produit obtenu se présente sous forme d'une crème blanche, douce et onctueuse utilisable dans le domaine cosmétique et/ou dermatologique selon la nature des actifs (de surface et de profondeur) choisis.

On donne ci-après des exemples particuliers de composition cosmétique conforme à l'invention. Les compositions sont données en % pondéral.

### EXEMPLE 1 : Crème double liposomes, dépigmentante

- *Vésicules de profondeur :*

| | |
|---|---|
| A/cholestérol/ dicétylphosphate dans un rapport pondéral 47,5/47,5/5 | 3 % |
| Acide kojique (actif) | 0,5 % |

- *Vésicules de surface :*

| | |
|---|---|
| D/ dimyristylphosphate dans un rapport pondéral 95/5 | 3 % |
| Acide salicylique (actif) | 0,3 % |

- *Phase grasse :*

| | |
|---|---|
| Huiles végétales ou minérales | 10 % |
| Huile de silicone | 5 % |
| Acide caféique (actif) | 0,7 % |

- *Phase aqueuse :*

| | |
|---|---|
| Glycérol | 3 % |
| Polymère carboxyvinylique (gélifiant) | 0,4 % |
| Triéthanolamine | qsp pH = 6 |
| Eau déminéralisée | qsp 100 % |

### EXEMPLE 2 : Crème double liposomes, dépigmentante

Cette crème se différencie de celle de l'exemple 1 par l'emploi de l'acide n-octanoyl-5 salicylique au lieu de l'acide salicylique comme actif de surface.

### EXEMPLE 3 : Crème double liposomes, antirides

- *Vésicules de profondeur :*

| | |
|---|---|
| Stéarate de PEG 8/cholestérol/acylglutamate de sodium dans un rapport pondéral 47,5/47,5/5 | 3 % |
| Collagène soluble (actif) | 1 % |
| Protéine hydrolysée de soja (actif) | 0,1 % |

- *Vésicules de surface :*

| | |
|---|---|
| Natipide II commercialisé par la Société Natterman | 3 % |
| Acétate de tocophéryle (actif) | 0,5 % |
| Glycérine (actif) | 4,5 % |
| Lactate de sodium (actif) | 0,35 % |

- *Phase grasse :*

| | |
|---|---|
| Huiles végétales | 13 % |
| Beurre de karité | 4 % |
| Cyclométhicone | 4 % |

- *Phase aqueuse :*

| | |
|---|---|
| Conservateur | 1,3 % |
| Parfum | 0,4 % |
| Polymère carboxyvinylique (gélifiant) | 0,4 % |
| Soude | qsp pH = 6 |
| Eau | qsp 100 % |

## Revendications

1. Composition cosmétique ou dermatologique pour le traitement simultané des couches superficielles et profondes de la peau, caractérisée en ce qu'elle comprend une première dispersion de vésicules lipidiques aptes à pénétrer dans les couches profondes de la peau et contenant au moins un premier actif apte à traiter ces couches profondes et une deuxième dispersion de vésicules lipidiques aptes à pénétrer dans les couches superficielles de la peau et contenant au moins un second actif apte à traiter ces couches superficielles, à condition que si les actifs procurent des effets différents, a) les vésicules de la première dispersion ne soient pas à base de lécithine d'oeuf hydrogénée, de cholestérol et de dicétylphosphate lorsque les vésicules de la deuxième dispersion sont à base de lécithine de soja, de cholestérol et de dicétylphosphate ou b) les vésicules de la première dispersion ne soient pas à base d'oléate de diglycérol, de cholestérol et de dicétylphosphate lorsque les vésicules de la deuxième dispersion sont à base de cétyléther de triglycéryle, de cholestérol et de dicétylphosphate, et à condition que :
- si la composition est une composition hydratante et qu'un premier actif est choisi parmi les polyols, les sucres, les protides, les vitamines et leurs dérivés, les céramides, les acides gras essentiels et les stérols, un second actif ne soit pas choisi parmi les polyols, les dérivés azotés d'acide carboxylique, les agents kératolytiques, les sels inorganiques, les lipoaminoacides :
- si la composition est une composition antiacnéique et qu'un premier actif est choisi parmi les agents antimicrobiens, antiseptiques, antibiotiques, anti-inflammatoires, antiséborrhéïques, le rétinol et ses dérivés, un second actif ne soit pas choisi parmi les agents kératolytiques, protecteurs, hydratants et antioxydants ;
- si la composition est une composition amincissante et qu'un premier actif est choisi parmi les agents lipolytiques, drainants et raffermissants, un second actif ne soit pas choisi parmi les agents de traitement de surface cutanée, d'exfoliation, de lissage et d'adoucissement de la peau ;
- si la composition est une composition pour lutter contre le vieillissement et qu'un premier actif est choisi parmi les agents hydratants, antiradicaux libres, kératolytiques, les protides, les agents antiélastase et anticollagénase, les enzymes, les dérivés d'acide gras, un second actif ne soit pas choisi parmi les agents kératolytiques, tenseurs, hydratants, restructurants de surface, antiradicaux libres ;
- si la composition est une composition protectrice, nutritive et/ou raffermissante et qu'un premier actif est choisi parmi les agents anti-radicaux libres oxygénés, les protides, les sucres, les enzymes, les vitamines, les nucléotides et leurs enchaînements, les oligoéléments, les coenzymes, les phospholipides riches en acides gras insaturés, les dérivés du silicium et les extraits végétaux, un second actif ne soit pas choisi parmi les filtres, les agents anti-radicaux libres oxygénés, les sucres, les acides aminés, les vitamines, les céramides, les produits insaponifiables issus d'huiles et les composants de la sueur ;
- si un premier actif est choisi parmi les accepteurs de spin, un second actif ne soit pas choisi parmi les filtres antisolaires ;
- si la composition est une composition dépigmentante et qu'un premier actif est choisi parmi les agents antipigmentants, dépigmentants et inhibiteurs de la tyrosinase, un second actif ne soit pas choisi parmi les agents kératolytiques, hydratants et protecteurs.

2. Composition cosmétique ou dermatologique pour le traitement simultané des couches superficielles et profondes de la peau, caractérisée en ce qu'elle comprend une première dispersion de vésicules lipidiques aptes à pénétrer dans les couches profondes de la peau et contenant au moins un premier actif apte à traiter ces couches profondes et une deuxième dispersion de vésicules lipidiques aptes à pénétrer dans les couches superficielles de la peau et contenant au moins un second actif apte à traiter ces couches superficielles, à condition que si les actifs procurent des effets différents, a) les vésicules de la première dispersion ne soient pas à base de lécithine d'oeuf hydrogénée, de cholestérol et de dicétylphosphate lorsque les vésicules de la deuxième dispersion sont à base de lécithine de soja, de cholestérol et de dicétylphosphate ou b) les vésicules de la première dispersion ne soient pas à base d'oléate de diglycérol, de cholestérol et de dicétylphosphate lorsque les vésicules de la deuxième dispersion sont à base de cétyléther de triglycéryle, de cholestérol et de dicétylphosphate, et à condition que :
- si un premier actif est choisi parmi les polyols, les sucres, les protides, les vitamines et leurs dérivés, les céramides, les acides gras essentiels et les stérols, un second actif ne soit pas choisi parmi les polyols, les dérivés azotés d'acide carboxylique, les agents kératolytiques, les sels inorganiques, les lipoaminoacides ;
- si un premier actif est choisi parmi les agents antimicrobiens, antiseptiques, antibiotiques, anti-inflammatoires, antiséborrhéïques, le rétinol et ses dérivés, un second actif ne soit pas choisi parmi les agents kératolytiques, protecteurs, hydratants et antioxydants ;
- si un premier actif est choisi parmi les agents lipolytiques, drainants et raffermissants, un second actif ne soit pas choisi parmi les agents de traitement de surface cutanée, d'exfoliation, de lissage et d'adoucissement de la peau ;
- si un premier actif est choisi parmi les agents hydratants, antiradicaux libres, kératolytiques, les protides, les agents antiélastase et anticollagénase, les enzymes, les dérivés d'acide gras, un second actif ne soit pas choisi parmi les agents kératolytiques, tenseurs, hydratants, restructurants de surface, antiradicaux libres ;
- si un premier actif est choisi parmi les agents anti-radicaux libres oxygénés, les protides, les sucres, les enzymes, les vitamines, les nucléotides et leurs enchaînements, les oligoéléments, les coenzymes, les phospholipides riches en acides gras insaturés, les dérivés du silicium et les extraits végétaux, un second actif ne soit pas choisi parmi les filtres, les agents anti-radicaux libres oxygénés, les sucres, les acides aminés, les vitamines, les céramides, les produits insaponifiables issus d'huiles et les composants de la sueur ;
- si un premier actif est choisi parmi les accepteurs de spin, un second actif ne soit pas choisi parmi les filtres antisolaires ;
- si un premier actif est choisi parmi les agents antipigmentants, dépigmentants et inhibiteurs de la tyrosinase, un second actif ne soit pas choisi parmi les agents kératolytiques, hydratants et protecteurs.

3. Composition selon la revendication 1 ou 2, caractérisée en ce que les vésicules de la première dispersion assurent une diffusion d'ASL (iodure de N-(1-oxyl-2,2,6,6-tétraméthyl-4-pipéridinyl)-N-diméthyl-N-hydroxyéthylammonium], dans le *Stratum Corneum*, > 1.10⁻⁷ cm² /s et en ce que les vésicules de la seconde dispersion assurent une diffusion d'ASL dans le *Stratum Corneum* < 1.10⁻⁷ cm² /s.

4. Composition l'une quelconque des revendications 1 à 3, caractérisée en ce que les vésicules de la première dispersion sont à l'état fluide à température ambiante et les vésicules de la seconde dispersion sont à l'état gélifié à température ambiante.

5. Composition selon l'une quelconque des revendications 1 à 4, caractérisée en ce que les vésicules de la première dispersion assurent une encapsulation du glucose pendant au moins 24 heures et en ce que les vésicules de la seconde dispersion assurent une encapsulation du glucose pendant moins de 24 heures.

6. Composition selon l'une quelconque des revendications 1 à 5, caractérisée en ce que les vésicules de la première dispersion sont formées de lipides comportant au moins une chaîne grasse linéaire et saturée ayant de 16 à 30 atomes de carbone.

7. Composition selon l'une quelconque des revendications 1 à 6, caractérisée en ce que les vésicules de la première dispersion sont formées d'au moins un lipide choisi parmi les phospholipides naturels hydrogénés, les phospholipides synthétiques saturés, les alkyléthers de polyols à au moins une chaîne linéaire grasse, les alkylesters de polyols à au moins une chaîne grasse, et leurs mélanges.

8. Composition selon l'une quelconque des revendications 1 à 7, caractérisée en ce que les vésicules de la première dispersion sont formées d'au moins un lipide choisi parmi :
Cétyléther de triglycéryle / cholestérol / lipoaminoacide caséique ;
Mélange de mono-, di- et tri-cétyléther de triglycéryle / cholestérol / dicétylphosphate ; Cétyléther de triglycéryle / cholestérol / dicétylphosphate ;
Sorbitan palmitate / cholestérol / acylglutamate de sodium ;
Stéarate de PEG 8 / cholestérol / acylglutamate de sodium ;
Distéarate de diglycéryle / cholestérol / acyglutamate de sodium ;
Mono et distéarate de saccharose / cholestérol / acylglutamate de sodium ;
Stéarate de PEG 8 / cholestérol / phytanetriol / acylglutamate de sodium ;
Distéarate de méthylglucose polyoxyéthyléné à 20 moles d'oxyde d'éthylène / cholestérol / acylglutamate de sodium ;
Lécithine hydrogénée / phytostérol polyoxyéthyléné ;
Tristéarate de tétraglycéryle / cholestérol / acylglutamate de sodium.

9. Composition selon l'une quelconque des revendications 1 à 8, caractérisée en ce que les vésicules de la seconde dispersion sont formées de lipides choisis parmi les phospholipides ioniques naturels contenant des chaînes grasses insaturées ayant de 16 à 30 atomes de carbone, les alkyléthers ou alkylesters de polyols comportant une ou plusieurs chaînes grasses par molécule, dont au moins une chaîne grasse de longueur inférieure à 16 atomes de carbone.

10. Composition selon l'une quelconque des revendications 1 à 9, caractérisée en ce que les vésicules de la seconde dispersion sont formées d'au moins un lipide choisi parmi :
Lécithine de tournesol ;
Lécithine de soja / éthanol / eau ;
Lécithine de soja / cholestérol / propylène glycol ;
Ether de lauryl polyglycéryl-6-cétéarylglycol / dimyristylphosphate.

11. Composition selon l'une quelconque des revendications 1 à 10, caractérisée en ce que l'actif de la première dispersion et celui de la seconde dispersion assurent la même fonction et/ou le même type d'effet.

12. Composition selon l'une quelconque des revendications 1 à 11, caractérisée en ce que les actifs de première et seconde dispersion sont les mêmes.

13. Composition selon l'une quelconque des revendications 1 à 12, caractérisée en ce que l'actif contenu dans la première dispersion est choisi parmi les agents précurseurs de la biosynthèse des protéines structurales, les agents antimycosiques, antiradicaux libres O₂^{·}, les antioxygènes singulets et OH^{·}, ₁O₂^{·}, OH^{·} et ROO^{·} des peptides, des vitamines, des acides aminés, des agents protecteurs vasculaires.

14. Composition selon l'une quelconque des revendications 1 à 13, caractérisée en ce que l'actif contenu dans la première dispersion est choisi parmi les agents antimycosiques et les agents protecteurs vasculaires.

15. Composition selon l'une quelconque des revendications 1 à 14, caractérisée en ce que l'actif contenu dans la seconde dispersion est choisi parmi les agents hydratants, kératolytiques, émollients, humectants, restructurants de surface, exfoliants, antiradicaux libres ROO^{·}, les antioxygènes singulets, les filtres solaires, les sucres et les acides gras, les α hydroxy-acides, les phopholipides extraits de poulpe.

16. Composition selon l'une quelconque des revendications 1 à 15, caractérisée en ce qu'elle contient en outre une phase huileuse dispersée dans une phase aqueuse.

17. Composition selon l'une quelconque des revendications 1 à 16, caractérisée en ce qu'elle contient, en outre, des adjuvants hydrophiles.

18. Composition selon l'une quelconque des revendications 1 à 17, caractérisée en ce que les vésicules de la première dispersion et/ou celles de la seconde dispersion renferment de l'ASL.

19. Utilisation de la composition selon l'une quelconque des revendications 1 à 18 pour un traitement de la peau, choisi parmi les traitements antiâge, antirides, dépigmentants, nutritifs, amincissants, hydratants et antiacnéïques.

20. Utilisation de la composition selon l'une quelconque des revendications 1 à 17, pour la préparation d'une pommade destinée au traitement des maladies de peau.

21. Utilisation selon la revendication 20, caractérisée en ce que la pommade est destinée au traitement antimycosique, antidermite ou antipsoriasis de la peau.

22. Procédé de traitement cosmétique de la peau, caractérisé en ce qu'il consiste à appliquer sur la peau une composition selon l'une quelconque des revendications 1 à 18.

## Claims

1. Cosmetic or dermatological composition for the simultaneous treatment of the surface and deep layers of the skin, characterized in that it includes a first dispersion of lipid vesicles capable of entering the deep layers of the skin and containing at least one first active substance capable of treating these deep layers, and a second dispersion of lipid vesicles capable of entering the surface layers of the skin and containing at least one second active substance capable of treating these surface layers, on condition that if the active substances provide different effects, a) the vesicles of the first dispersion are not based on hydrogenated egg lecithin, cholesterol and dicetyl phosphate when the vesicles of the second dispersion are based on soya lecithin, cholesterol and dicetyl phosphate, or b) the vesicles of the first dispersion are not based on diglycerol oleate, cholesterol and dicetyl phosphate when the vesicles of the second dispersion are based on triglyceryl cetyl ether, cholesterol and dicetyl phosphate, and on condition that:
- if the composition is a hydrating composition and if a first active substance is chosen from polyols, sugars, proteins, vitamins and their derivatives, ceramides, essential fatty acids and sterols, a second active substance is not chosen from polyols, nitrogen derivatives of carboxylic acid, keratolytic agents, inorganic salts and lipoaminoacids;
- if the composition is an antiacne composition and if a first active substance is chosen from antimicrobial, antiseptic, antibiotic, antiinflammatory, antiseborrhoeic agents, retinol and its derivatives, a second active substance is not chosen from keratolytic, protective, hydrating and antioxidant agents;
- if the composition is a slimming composition and if a first active substance is chosen from lipolytic, draining and firming-up agents, a second active substance is not chosen from the agents for the treatment of cutaneous surface, for exfoliation, for smoothing and for softening the skin;
- if the composition is a composition for combating aging and if a first active substance is chosen from hydrating, anti-free radical and keratolytic agents, proteins, antielastase and anticollagenase agents, enzymes and fatty acid derivatives, a second active substance is not chosen from keratolytic, tensor, hydrating, surface restructuring and anti-free radical agents;
- if the composition is a protective, nutritive and/or firming-up composition and if a first active substance is chosen from the agents against oxygenated free radicals, proteins, sugars, enzymes, vitamins, nucleotides and their chain sequences, oligoelements, coenzymes, phospholipids rich in unsaturated fatty acids, silicon derivatives and plant extracts, a second active substance is not chosen from filters, agents against oxygenated free radicals, sugars, amino acids, vitamins, ceramides, nonsaponifiable products originating from oils and the components of sweat;
- if a first active substance is chosen from spin-acceptors, a second active substance is not chosen from sunscreens;
- if the composition is a depigmenting composition and if a first active substance is chosen from antipigmenting or depigmenting agents and tyrosinase inhibitors, a second active agent is not chosen from keratolytic, hydrating and protecting agents.

2. Cosmetic or dermatological composition for the simultaneous treatment of the surface and deep layers of the skin, characterized in that it includes a first dispersion of lipid vesicles capable of entering the deep layers of the skin and containing at least one first active substance capable of treating these deep layers, and a second dispersion of lipid vesicles capable of entering the surface layers of the skin and containing at least one second active substance capable of treating these surface layers, on condition that if the active substances provide different effects, a) the vesicles of the first dispersion are not based on hydrogenated egg lecithin, cholesterol and dicetyl phosphate when the vesicles of the second dispersion are based on soya lecithin, cholesterol and dicetyl phosphate, or b) the vesicles of the first dispersion are not based on diglycerol oleate, cholesterol and dicetyl phosphate when the vesicles of the second dispersion are based on triglyceryl cetyl ether, cholesterol and dicetyl phosphate, and on condition that:
- if a first active substance is chosen from polyols, sugars, proteins, vitamins and their derivatives, ceramides, essential fatty acids and sterols, a second active substance is not chosen from polyols, nitrogen derivatives of carboxylic acid, keratolytic agents, inorganic salts and lipoaminoacids;
- if a first active substance is chosen from antimicrobial, antiseptic, antibiotic, antiinflammatory and antiseborrhoeic agents, retinol and its derivatives, a second active substance is not chosen from keratolytic, protecting, hydrating and antioxidant agents;
- if a first active substance is chosen from lipolytic, draining and firming-up agents, a second active substance is not chosen from agents for the treatment of cutaneous surface, for exfoliation, for smoothing and for softening the skin;
- if a first active substance is chosen from hydrating, anti-free radical and keratolytic agents, proteins, antielastase and anticollagenase agents, enzymes, fatty acid derivatives, a second active substance is not chosen from keratolytic, tensor, hydrating, surface-restructuring and anti-free radical agents;
- if a first active substance is chosen from agents against oxygenated free radicals, proteins, sugars, enzymes, vitamins, nucleotides and their chain sequences, oligoelements, coenzymes, phospholipids which are rich in unsaturated fatty acids, silicon derivatives and plant extracts, a second active substance is not chosen from filters, agents against oxygenated free radicals, sugars, amino acids, vitamins, ceramides, nonsaponifiable products originating from oils and the components of sweat;
- if a first active substance is chosen from spin-acceptors, a second active substance is not chosen from sunscreens;
- if a first active substance is chosen from antipigmenting or depigmenting agents and tyrosinase inhibitors, a second active substance is not chosen from keratolytic, hydrating and protecting agents.

3. Composition according to Claim 1 or 2, characterized in that the vesicles of the first dispersion ensure a distribution of ASL [N-(1-oxido-2,2,6,6-tetramethyl-4-piperidyl)-N-dimethyl-N-hydroxyethylammonium iodide] in the *stratum corneum* > 1×10⁻⁷ cm²/s and in that the vesicles of the second dispersion ensure a distribution of ASL in the *stratum corneum* < 1×10⁻⁷ cm²/s.

4. Composition according to any one of Claims 1 to 3, characterized in that the vesicles of the first dispersion are in the fluid state at ambient temperature and the vesicles of the second dispersion are in the gelled state at ambient temperature.

5. Composition according to any one of Claims 1 to 4, characterized in that the vesicles of the first dispersion ensure an encapsulation of glucose for at least 24 hours and in that the vesicles of the second dispersion ensure an encapsulation of glucose for less than 24 hours.

6. Composition according to any one of Claims 1 to 5, characterized in that the vesicles of the first dispersion are made up of lipids comprising at least one linear and saturated fatty chain containing from 16 to 30 carbon atoms.

7. Composition according to any one of Claims 1 to 6, characterized in that the vesicles of the first dispersion are made up of at least one lipid chosen from hydrogenated natural phospholipids, saturated synthetic phospholipids, polyol alkyl ethers containing at least one fatty linear chain, polyol alkyl esters containing at least one fatty chain, and mixtures thereof.

8. Composition according to any one of Claims 1 to 7, characterized in that the vesicles of the first dispersion are made up of at least one lipid chosen from: Triglyceryl cetyl ether/cholesterol/casein lipoaminoacid; Mixture of triglyceryl mono-, di- and tricetyl ether/cholesterol/dicetyl phosphate;
Triglyceryl cetyl ether/cholesterol/dicetyl phosphate;
Sorbitan palmitate/cholesterol/sodium acylglutamate;
PEG 8 stearate/cholesterol/sodium acylglutamate;
Diglyceryl distearate/cholesterol/sodium acylglutamate;
Sucrose mono- and distearate/cholesterol/sodium acylglutamate;
PEG 8 stearate/cholesterol/phytanetriol/sodium acylglutamate;
Methyl glucose distearate polyoxyethylenated with 20 moles of ethylene oxide/cholesterol/sodium acylglutamate; Hydrogenated lecithin/polyoxyethylenated phytosterol; Tetraglyceryl tristearate/cholesterol/sodiumacylglutamate.

9. Composition according to any one of Claims 1 to 8, characterized in that the vesicles of the second dispersion are made up of lipids chosen from natural ionic phospholipids containing unsaturated fatty chains containing from 16 to 30 carbon atoms, polyol alkyl ethers or alkyl esters containing one or a number of fatty chains per molecule, including at least one fatty chain of length shorter than 16 carbon atoms.

10. Composition according to any one of Claims 1 to 9, characterized in that the vesicles of the second dispersion are made up of at least one lipid chosen from :
Sunflower lecithin;
Soya lecithin/ethanol/water;
Soya lecithin/cholesterol/propylene glycol;
Polyglyceryl-6-cetearyl glycol lauryl ether/dimyristyl phosphate.

11. Composition according to any one of Claims 1 to 10, characterized in that the active substance of the first dispersion and that of the second dispersion provide the same function and/or the same type of effect.

12. Composition according to any one of Claims 1 to 11, characterized in that the active substances of the first and second dispersion are the same.

13. Composition according to any one of Claims 1 to 12, characterized in that the active substance present in the first dispersion is chosen from precursor agents for the biosynthesis of structural proteins, antimycotic agents, agents against free O₂^{·} radicals, against singlet oxygens and OH^{·}, ₁O₂^{·}, OH^{·} and ROO^{·}, peptides, vitamins, amino acids and vascular protecting agents.

14. Composition according to any one of Claims 1 to 13, characterized in that the active substance present in the first dispersion is chosen from antimycotic agents and vascular protecting agents.

15. Composition according to any one of Claims 1 to 14, characterized in that the active substance present in the second dispersion is chosen from hydrating, keratolytic, emollient, moisturizing, surface-restructuring and exfoliating agents, agents against free ROO^{·} radicals, against singlet oxygens, sunscreens, sugars and fatty acids, α-hydroxyacids and phospholipids extracted from octopus.

16. Composition according to any one of Claims 1 to 15, characterized in that it additionally contains an oily phase dispersed in an aqueous phase.

17. Composition according to any one of Claims 1 to 16, characterized in that it additionally contains hydrophilic adjuvants.

18. Composition according to any one of Claims 1 to 17, characterized in that the vesicles of the first dispersion and/or those of the second dispersion contain ASL.

19. Use of the composition according to any one of Claims 1 to 18 for a treatment of the skin, chosen from the antiaging, antiwrinkle, depigmenting, nutrient, slimming, hydrating and antiacne treatments.

20. Use of the composition according to any one of Claims 1 to 17, for the preparation of an ointment intended for the treatment of skin diseases.

21. Use according to Claim 20, characterized in that the ointment is intended for the treatment of the skin against mycosis, dermatitis or psoriasis.

22. Process for cosmetic treatment of the skin, characterized in that it consists in applying to the skin a composition according to any one of Claims 1 to 18.

## Patentansprüche

1. Kosmetische oder dermatologische Zusammensetzung zur gleichzeitigen Behandlung der oberflächlichen und der unteren Hautschichten, dadurch gekennzeichnet, daß sie eine erste Dispersion von Lipidvesikeln, die in die unteren Hautschichten eindringen können und mindestens einen ersten zur Behandlung der unteren Hautschichten geeigneten Wirkstoff enthalten, und eine zweite Dispersion von Lipidvesikeln, die in die oberflächlichen Hautschichten eindringen können und mindestens einen zweiten zur Behandlung der oberflächlichen Hautschichten geeigneten Wirkstoff enthalten, enthält, mit der Maßgabe, daß, wenn die Wirkstoffe unterschiedliche Wirkungen hervorrufen, (a) die Vesikel der ersten Dispersion nicht auf der Basis von hydriertem Lecithin aus Eiern, Cholesterin und Dicetylphosphat vorliegen, wenn die Vesikel der zweiten Dispersion auf der Basis von Lecithin aus Soja, Cholesterin und Dicetylphosphat vorliegen, oder (b) die Vesikel der ersten Dispersion nicht auf der Basis von Diglyceryloleat, Cholesterin und Dicetylphosphat vorliegen, wenn die Vesikel der zweiten Dispersion auf der Basis von Triglycerylcetylether, Cholesterin und Dicetylphosphat vorliegen, und mit der Maßgabe, daß
- wenn die Zusammensetzung eine hydratisierende Zusammensetzung ist und ein erster Wirkstoff unter Polyolen, Zuckern, Protidverbindungen (Proteinen, Peptiden und ihre Hydrolysaten), Vitaminen und ihren Derivaten, Ceramiden, essentiellen Fettsäuren und Sterinen ausgewählt ist, ein zweiter Wirkstoff nicht unter Polyolen, stickstoffhaltigen Carbonsäurederivaten, Keratolytika, anorganischen Salzen und Lipoaminosäuren ausgewählt ist;
- wenn die Zusammensetzung eine Zusammensetzung gegen Akne ist und ein erster Wirkstoff unter antimikrobiellen, antiseptischen, antibiotischen, entzündungshemmenden Mitteln, Mittel gegen Seborrhoe, Retinol und seinen Derivaten ausgewählt ist, ein zweiter Wirkstoff nicht unter Keratolytika, Schutzmitteln, Hydratisierungsmittel und Antioxidantien ausgewählt ist;
- wenn die Zusammensetzung eine schlankermachende Zusammensetzung ist und ein erster Wirkstoff unter fettabbauenden, entwässernden und straffenden Mitteln ausgewählt ist, ein zweiter Wirkstoff nicht unter Mitteln zur Behandlung der Hautoberfäche, Exfoliationsmitteln, glättenden Mitteln und reizlindernden Mitteln ausgewählt ist;
- wenn die Zusammensetzung eine Zusammensetzung zur Bekämpfung der Alterung ist und ein erster Wirkstoff unter Hydratisierungsmitteln, Mitteln gegen freie Radikale, Keratolytika, Protidverbindungen, Mitteln gegen Elastase und Kollagenase, Enzymen und Fettsäurederivaten ausgewählt ist, ein zweiter Wirkstoff nicht unter Keratolytika, Tensiden, Hydratisierungsmitteln, Mitteln zur Restrukturierung der Oberfläche und Mitteln gegen freie Radikale ausgewählt ist;
- wenn die Zusammensetzung eine schützende, nährende oder straffende Zusammensetzung ist und ein erster Wirkstoff unter Mitteln gegen freie Sauerstoffradikale, Protidverbindungen, Zuckern, Enzymen, Vitaminen, Nucleotiden und ihren Verknüpfungen, Spurenelementen, Coenzymen, Phosphlipiden mit hohem Anteil an ungesättigten Fettsäuren, Siliciumderivaten und Pflanzenextrakten ausgewählt ist, ein zweiter Wirkstoff nicht unter Filtern, Mitteln gegen freie Sauerstoffradikale, Zuckern, Aminosäuren, Vitaminen, Ceramiden, von Ölen abgeleiteten unverseifbaren Produkten und Schweißbestandteilen ausgewählt ist;
- wenn ein erster Wirkstoff unter Spin-Akzeptoren ausgewählt ist, ein zweiter Wirkstoff nicht unter Sonnenschutzfiltern ausgewählt ist; und
- wenn die Zusammensetzung eine depigmentierende Zusammensetzung ist und ein erster Wirkstoff unter Mitteln gegen Pigmentbildung, depigmentierenden Mitteln und Tyrosinase-Inhibitoren ausgewählt ist, ein zweiter Wirkstoff nicht unter Keratolytika, Hydratisierungsmitteln und Schutzmitteln ausgewählt ist.

2. Kosmetische oder dermatologische Zusammensetzung zur gleichzeitigen Behandlung der oberflächlichen und der unteren Hautschichten, dadurch gekennzeichnet, daß sie eine erste Dispersion von Lipidvesikeln, die in die unteren Hautschichten eindringen können und mindestens einen ersten zur Behandlung der unteren Hautschichten geeigneten Wirkstoff enthalten, und eine zweite Dispersion von Lipidvesikeln, die in die oberflächlichen Hautschichten eindringen können und mindestens einen zweiten zur Behandlung der oberflächlichen Hautschichten geeigneten Wirkstoff enthalten, enthält, mit der Maßgabe, daß, wenn die Wirkstoffe unterschiedliche Wirkungen hervorrufen, (a) die Vesikel der ersten Dispersion nicht auf der Basis von hydriertem Lecithin aus Eiern, Cholesterin und Dicetylphosphat vorliegen, wenn die Vesikel der zweiten Dispersion auf der Basis von Lecithin aus Soja, Cholesterin und Dicetylphosphat vorliegen, oder (b) die Vesikel der ersten Dispersion nicht auf der Basis von Diglyceryloleat, Cholesterin und Dicetylphosphat vorliegen, wenn die Vesikel der zweiten Dispersion auf der Basis von Triglycerylcetylether, Cholesterin und Dicetylphosphat vorliegen, und mit der Maßgabe, daß
- wenn ein erster Wirkstoff unter Polyolen, Zuckern, Protidverbindungen, Vitaminen und ihren Derivaten, Ceramiden, essentiellen Fettsäuren und Sterinen ausgewählt ist, ein zweiter Wirkstoff nicht unter Polyolen, stickstoffhaltigen Carbonsäurederivaten, Keratolytika, anorganischen Salzen und Lipoaminosäuren ausgewählt ist;
- wenn ein erster Wirkstoff unter antimikrobiellen, antiseptischen, antibiotischen, entzündungshemmenden Mitteln, Mittel gegen Seborrhoe, Retinol und seinen Derivaten ausgewählt ist, ein zweiter Wirkstoff nicht unter Keratolytika, Schutzmitteln, Hydratisierungsmittel und Antioxidantien ausgewählt ist;
- wenn ein erster Wirkstoff unter fettabbauenden, entwässernden und straffenden Mitteln ausgewählt ist, ein zweiter Wirkstoff nicht unter Mitteln zur Behandlung der Hautoberfäche, Exfoliationsmitteln, glättenden Mitteln und reizlindernden Mitteln ausgewählt ist;
- wenn ein erster Wirkstoff unter Hydratisierungsmitteln, Mitteln gegen freie Radikale, Keratolytika, Protidverbindungen, Mitteln gegen Elastase und Kollagenase, Enzymen und Fettsäurederivaten ausgewählt ist, ein zweiter Wirkstoff nicht unter Keratolytika, Tensiden, Hydratisierungsmitteln, Mitteln zur Restrukturierung der Oberfläche und Mitteln gegen freie Radikale ausgewählt ist;
- wenn ein erster Wirkstoff unter Mitteln gegen freie Sauerstoffradikale, Protidverbindungen, Zuckern, Enzymen, Vitaminen, Nucleotiden und ihren Verknüpfungen, Spurenelementen, Coenzymen, Phospholipiden mit hohem Anteil an ungesättigten Fettsäuren, Siliciumderivaten und Pflanzenextrakten ausgewählt ist, ein zweiter Wirkstoff nicht unter Filtern, Mitteln gegen freie Sauerstoffradikale, Zuckern, Aminosäuren, Vitaminen, Ceramiden, von Ölen abgeleiteten unverseifbaren Produkten und Schweißbestandteilen ausgewählt ist;
- wenn der erste Wirkstoff unter Spin-Akzeptoren ausgewählt ist, der zeite Wirkstoff nicht unter Sonnenschutzfiltern ausgewählt ist; und
- wenn ein erster Wirkstoff unter Mitteln gegen Pigmentbildung, depigmentierenden Mitteln und Tyrosinase-Inhibitoren ausgewählt ist, ein zweiter Wirkstoff nicht unter Keratolytika, Hydratisierungsmitteln und Schutzmitteln ausgewählt ist.

3. Zusammensetzung nach Anspruch 1 oder 2, dadurch gekennzeichnet, daß die Vesikel der ersten Dispersion eine Diffusionskonstante der Diffusion von ASL (N-(1-Oxyl2,2,6,6-tetramethylpiperidin-4-yl)-N-dimethyl-N-hydroxyethylammoniumjodid) in das *Stratum corneum* von > 1·10⁻⁷ cm² s⁻¹ und die Vesikel der zweiten Dispersion eine ASL-Diffusion in das *Stratum corneum* von < 1·10⁻⁷ cm² s⁻¹ gewährleisten.

4. Zusammensetzung nach einem der Ansprüche 1 bis 3, dadurch gekennzeichnet, daß die Vesikel der ersten Dispersion bei Raumtemperatur in einem flüssigen Zustand und die Vesikel der zweiten Dispersion bei Raumtemperatur im Gelzustand vorliegen.

5. Zusammensetzung nach einem der Ansprüche 1 bis 4, dadurch gekennzeichnet, daß die Vesikel der ersten Dispersion einen Einschluß von Glucose während mindestens 24 Stunden und die Vesikel der zweiten Dispersion einen Einschluß von Glucose während weniger als 24 Stunden gewährleisten.

6. Zusammensetzung nach einem der Ansprüche 1 bis 5, dadurch gekennzeichnet, daß die Vesikel der ersten Dispersion aus Lipiden gebildet sind, die mindestens eine lineare und gesättigte Fettkette mit 16 bis 30 Kohlenstoffatomen aufweisen.

7. Zusammensetzung nach einem der Ansprüche 1 bis 6, dadurch gekennzeichnet, daß die Vesikel der ersten Dispersion aus mindestens einem Lipid gebildet sind, das unter hydrierten natürlichen Phospholipiden, gesättigten synthetischen Phospholipiden, Alkylethern von Polyolen mit mindestens einer geradkettigen Fettkette und Alkylestern von Polyolen mit mindestens einer Fettkette sowie ihren Gemischen ausgewählt ist.

8. Zusammensetzung nach einem der Ansprüche 1 bis 7, dadurch gekennzeichnet, daß die Vesikel der ersten Dispersion aus mindestens einem Lipid gebildet sind, das ausgewählt ist unter:
Triglycerylcetylether / Cholesterin / Casein-Lipoaminosäure;
einem Gemisch aus Triglycerylmono-, -di- und - tricetylether / Cholesterin / Dicetylphosphat;
Triglycerylcetylether / Cholesterin / Dicetylphosphat;
Sorbitanpalmitat / Cholesterin / Natriumacylglutamat;
PEG-8-Stearat / Cholesterin / Natriumacylglutamat;
Diglyceryldistearat / Cholesterin / Natriumacylglutamat;
Saccharosemono- und -distearat / Cholesterin / Natriumacylglutamat;
PEG-8-Stearat / Cholesterin / Phytantriol / Natriumacylglutamat;
Methylglucosedistearat, das mit 20 mol Ethylenoxid polyethoxyliert ist / Cholesterin / Natriumacylglutamat;
hydriertes Lecithin / polyethoxyliertes Phytosterin;
Tetraglyceryltristearat / Cholesterin / Natriumacylglutamat.

9. Zusammensetzung nach einem der Ansprüche 1 bis 8, dadurch gekennzeichnet, daß die Vesikel der zweiten Dispersion aus Lipiden gebildet sind, die unter natürlichen ionischen Phospholipiden, die ungesättigte Fettketten mit 16 bis 30 Kohlenstoffatomen enthalten, und Alkylethern oder Alkylestern von Polyolen mit einer oder mehreren Fettketten pro Molekül, wobei mindestens eine Fettkette weniger als 16 Kohlenstoffatome aufweist, ausgewählt ist.

10. Zusammensetzung nach einem der Ansprüche 1 bis 9, dadurch gekennzeichnet, daß die Vesikel der zweiten Dispersion aus mindestens einem Lipid gebildet sind, das ausgewählt ist unter:
Lecithin aus Sonnenblumen;
Lecithin aus Soja / Ethanol / Wasser;
Lecithin aus Soja / Cholesterin / Propylenglykol;
Laurylpolyglyceryl-6-cetearylglykolether / Dimyristylphosphat.

11. Zusammensetzung nach einem der Ansprüche 1 bis 10, dadurch gekennzeichnet, daß die Wirkstoffe der ersten Dispersion und der zweiten Dispersion die gleiche Wirksamkeit und/oder die gleiche Wirkungsart aufweisen.

12. Zusammensetzung nach einem der Ansprüche 1 bis 11, dadurch gekennzeichnet, daß die Wirkstoffe der ersten Dispersion und der zweiten Dispersion identisch sind.

13. Zusammensetzung nach einem der Ansprüche 1 bis 12, dadurch gekennzeichnet, daß der in der ersten Dispersion enthaltene Wirkstoff unter Vorläufermaterialien der Biosynthese von Strukturproteinen, Antimykotika, Mittel gegen freie Radikale O₂·, Mitteln gegen Singulett-Sauerstoff und OH·, O₂·, OH· und ROO·, Peptiden, Vitaminen, Aminosäuren und vaskularen Schutzmitteln ausgewählt ist.

14. Zusammensetzung nach einem der Ansprüche 1 bis 13, dadurch gekennzeichnet, daß der in der zweiten Dispersion enthaltene Wirkstoff unter Antimykotika und vaskularen Schutzmittel ausgewählt ist.

15. Zusammensetzung nach einem der Ansprüche 1 bis 14, dadurch gekennzeichnet, daß der in der zweiten Dispersion enthaltene Wirkstoff unter Hydratisierungsmitteln, Keratolytika, Softenern, Feuchthaltemitteln, Mitteln zur Restrukturierung der Oberfläche, Exfoliationsmitteln, Mitteln gegen freie Radikale ROO·, Mitteln gegen Singulett-Sauerstoff, Sonnenschutzfiltern, Zuckern, Fettsäuren, α-Hydroxysäuren und aus Polypen gewonnenen Phospholipiden ausgewählt ist.

16. Zusammensetzung nach einem der Ansprüche 1 bis 15, dadurch gekennzeichnet, daß sie ferner eine in einer wäßrigen Phase dispergierte Ölphase enthält.

17. Zusammensetzung nach einem der Ansprüche 1 bis 16, dadurch gekennzeichnet, daß sie ferner hydrophile Hilfsstoffe enthält.

18. Zusammensetzung nach einem der Ansprüche 1 bis 17, dadurch gekennzeichnet, daß die Vesikel der ersten Dispersion und/oder der zweiten Dispersion ASL einkapseln.

19. Verwendung der Zusammensetzung nach einem der Ansprüche 1 bis 18 für eine Behandlung der Haut, die unter einer Behandlung gegen Alterung, einer Behandlung gegen Falten, einer depigmentierender Behandlung, einer Behandlung zur Nutrition, einer schlankermachenden Behandlung, einer hydratisierenden Behandlung und einer Behandlung gegen Akne ausgewählt ist.

20. Verwendung der Zusammensetzung nach einem der Ansprüche 1 bis 17 zur Herstellung einer für die Behandlung von Hautkrankheiten bestimmten Salbe.

21. Verwendung nach Anspruch 20, dadurch gekennzeichnet, daß die Salbe für die Behandlung der Haut gegen Mykosen, Dermatosen und Psoriasis bestimmt ist.

22. Verfahren zur kosmetischen Behandlung der Haut, dadurch gekennzeichnet, daß es im Auftragen einer Zusammensetzung nach einem der Ansprüche 1 bis 18 auf die Haut besteht.
